(19) Europäisches Patentamt
European Patent Office
Office européen des brevets

(11) Numéro de publication: **0 005 422**
**B1**

# (12) FASCICULE DE BREVET EUROPEEN

(45) Date de publication du fascicule du brevet: **27.10.82**

(21) Numéro de dépôt: **79870011.8**

(22) Date de dépôt: **02.05.79**

(51) Int. Cl.³: **C 07 C 53/126,**
C 07 C 103/127,
A 61 K 31/185,
A 61 K 31/16

## (54) Dérivés d'acides oméga-dialkylalkanoiques, leurs procédés de préparation ainsi que leur utilisation en thérapeutique.

(30) Priorité: **03.05.78 GB 1757078**

(43) Date de publication de la demande:
**14.11.79 Bulletin 79/23**

(45) Mention de la délivrance du brevet:
**27.10.82 Bulletin 82/43**

(84) Etats contractants désignés:
**AT CH DE FR GB IT NL SE**

(56) Documents cités:
AU - A - 240 784
DE - C - 222 809
GB - A - 760 114

CHEMICAL ABSTRACTS, vol. 43, No. 15, 10 août 1949 no. 6164g—6165c
Columbus, Ohio, USA W. KEIL et al.: "Fats from fatty acids with odd numbers of carbon atoms"
CHEMICAL ABSTRACTS, vol. 82, no. 26, 30 juin 1975, no. 178866t page 555
Columbus, Ohio, USA C. COHEN-ADDAD et al.: "Crystal structures of dipropyl- and tripropylacetic acid derivatives' III. Diethylpropionamide and dipropylpropionamide"
EUROPEAN JOURNAL OF MEDICINAL CHEMISTRY, CHIMICA THERAPEUTICA, vol. X. sept.-oct, 1975, no. 5 pages 453—462

(73) Titulaire: **SANOFI, société anonyme**
**40, Avenue George V**
**F-75008 Paris (FR)**

(72) Inventeur: **Chignac, Michel**
**"Le Gand" Avenue du Lac**
**F-04200 Sisteron (FR)**
Inventeur: **Grain, Claude**
**Villa "Les Olivettes" La Croix du Sud**
**F-04290 Volonne (FR)**
Inventeur: **Jammot, Fernand**
**Les Eygatières**
**F-04200 Sisteron (FR)**
Inventeur: **Pigerol, Charles**
**Rue Carnot 8**
**F-93400 Saint-Ouen (FR)**
Inventeur: **Eymard, Pierre Luc**
**Rue de la Liberté 22**
**F-38600 Fontaine (FR)**
Inventeur: **Combet, Madeleine Epouse Broll**
**Domaine de Valetiére Le Fontanil**
**F-38120 St. Egreve (FR)**
Inventeur: **Ferrandes, Bernard**
**Rue Fantin Latour**
**F-38640 Claix (FR)**
Inventeur: **Taillandier, Georges Marcel**
**Allée de la Piat 7**
**F-38240 Meylan (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 005 422 B1

Courier Press, Leamington Spa, England.

(72) Inventeur: **Benoit,Guyod, Jean/Louis Alain**
**13 Avenue de Verdun**
**F-38240 Meylan (FR)**
Inventeur: **Boucherle, André Louis**
**Avenue Eygala 116**
**F-38700 La Tronche (FR)**

(74) Mandataire: **Kenny, John E. et al,**
**c/o S.A. Labaz N.V. 1, avenue de Béjar**
**B-1120 Bruxelles (BE)**

Dérivés d'acide ω-dialkylalkanoïque, leurs procédés de préparation ainsi que leur utilisation en thérapeutique

La présente invention se rapporte, d'une manière générale, à de nouveaux dérivés d'acide ω-dialkylalkanoïque doués d'activités pharmacologiques et biochimiques ainsi qu'aux medicaments les contenant.

L'invention se rapporte également au procédé de préparation de ces dérivés d'acide ω-dialkylalkanoïque.

Les composés actifs de l'invention peuvent être représentés par la formule générale:

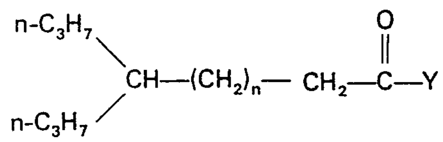
I

dans laquelle Y représente un radical $NH_2$ ou un radical $OM_{1/2}$ dans lequel M représente un atome de métal alcalino-terreux, par exemple un atome de calcium ou de magnésium, et n représente 0, 1 ou 2, à condition que lorsque Y représente $NH_2$, n représente 2.

Comme on le décrira plus en détail par la suite, on a découvert que les dérivés d'acide ω-dialkylalkanoïque de formule I possèdent, à des degrés divers, des activités biochimiques et pharmacologiques notamment des propriétés anticonvulsivante, antianoxique et inhibitrice compétitive de la transaminase γ-aminobutyrique α-cétoglutarique.

En conséquence, les composés de l'invention constitueront des agents particulièrement efficaces pour le traitement de différentes variétés de troubles neurologiques centraux consécutifs ou non à l'ischémie cérébrale ainsi que des troubles relevant plus particulièrement de la neuropsychiatrie.

Un autre objet de l'invention se rapporte, en conséquence, à de nouveaux médicaments utiles notamment pour le traitement de troubles neurologiques centraux consécutifs ou non à l'ischémie cérébrale par exemple l'épilepsie ainsi que des troubles relevant plus particulièrement de la neuropsychiatrie, médicaments dont le principe actif est constitué par un dérivé d'acide ω-dialkylalkanoïque de formule I.

L'administration journalière sera de préférence de 200 à 1500 mg de principe actif selon l'invention à un être humain de 60 kg quelle que soit la voie d'administration choisie.

Parmi les composés de formule I ci-dessus un certain nombre d'entre eux sont couverts de façon générale par le brevet britannique No 760.114 sans toutefois y avoir été cités. Tel est le cas notamment des sels de calcium ou de magnésium de l'acide n-propyl-3 hexanoïque qui doivent être considérés néanmoins comme de nouveaux produits.

Les dérivés de formule I pourront être préparés de différentes façons eu égard à leur structure chimique à savoir:

a) lorsque Y représente un radical $OM_{1/2}$ dans lequel M a la valeur indiquée en faisant réagir, dans un solvant approprié tel que l'eau, le n-propanol ou le benzène, un acide de formule générale:

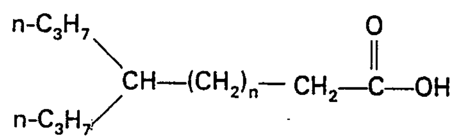
II

dans laquelle n a la même signification que précédemment, avec un oxyde de métal alcalino-terreux, par exemple l'oxyde de calcium ou de magnésium. La réaction aura lieu préférentiellement à une température comprise entre la température ambiante et 70°C.

b) lorsque Y représente un radical $NH_2$ en faisant réagir l'ammoniac avec le chlorure de n-propyl-5 octanoyle à une température comprise entre −15°C et 0°C et dans un milieu approprié, par exemple le toluène.

Quant aux acides de formule II, ceux-ci pourront être préparés suivant la méthode décrite dans le brevet français No 2.276.036.

Selon un autre procédé, ces acides de formule II peuvent être obtenus également comme suit:

a) lorsque n représente 0 ou 1, en hydrolysant un amide de formule générale:

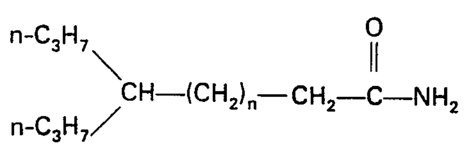
III

dans laquelle n représente 0 ou 1 et ce, au moyen d'une solution d'acide sulfurique à 80% en poids en présence de nitrite de sodium à une température comprise entre 55°C ± 5° pour obtenir l'acide désiré.

Ces amides de formule III peuvent être obtenus en hydrolysant, à une température comprise entre 80 et 85°C, un nitrile de formule générale:

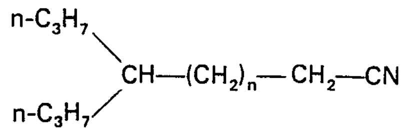

IV

dans laquelle n représente 0 ou 1, avec une solution d'acide sulfurique à 80% en poids, ce qui fournit les amides désirés de formule III.

Les nitriles de formule IV pourront être préparés au départ d'acides de formule générale:

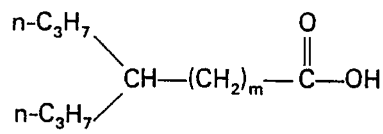

V

dans laquelle m représente 0 ou 1.

Ces acides de formule V sont d'abord transformés en leur chlorure d'acide par réaction avec le chlorure de thionyle dans le N,N-diméthylformamide à une température comprise entre la température ambiante et 70°C puis en estérifiant avec le méthanol, le chlorure obtenu et ce, à la température de reflux du milieu.

On réduit alors au reflux l'ester méthylique obtenu avec un mélange de borohydrure de potassium/chlorure de lithium et ce, dans le tétrahydrofuranne pour obtenir un alcool de formule générale:

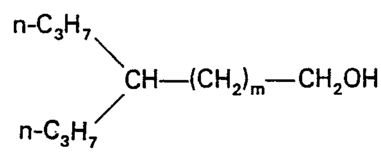

VI

dans laquelle m a la même signification que précédemment.

On fait ensuite réagir l'alcool de formule VI obtenu avec du benzènesulfochlorure à température ambiante et dans un accepteur d'acide par exemple la pyridine, ce qui fournit les dérivés benzènesulfonates de formule générale:

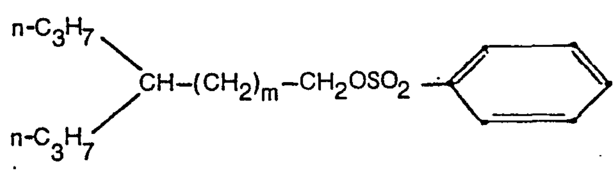

VII

dans laquelle m a la même signification que précédemment que l'on peut ensuite faire réagir avec le cyanure de sodium ou de potassium dans un solvent approprié, par exemple le diméthylsulfoxyde à une température comprise entre 70°C et 90°C pour obtenir les nitriles de formule IV.

b) lorsque n représente 2 ou 3, en faisant réagir un dérivé benzènesulfonate de formule VII dans laquelle m représente 1 ou 2, avec le malonate diéthylique en présence d'hydrure de sodium et dans un solvant polaire tel que le N,N-diméthylformamide pour obtenir un ester. On saponifie cet ester en présence d'hydroxyde de sodium ou de potassium pour obtenir un sel que l'on fait réagir avec un acide fort par exemple l'acide chlorhydrique pour obtenir le dérivé d'acide malonique de formule générale:

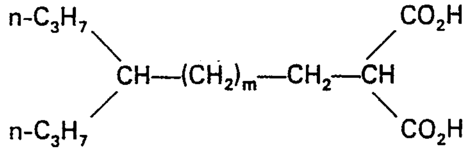

VIII)

dans laquelle m représente 1 ou 2, que l'on décarboxyle à une température comprise entre 150°C et 190°C pour obtenir l'acide désiré.

Les benzènesulfonates de formule VII dans laquelle m représente 2 peuvent être obtenus de la même manière que les autres dérivés de formule VII dans laquelle m représente 0 ou 1.

Il est connu que la plupart des troubles du système nerveux central tels que l'épilepsie constituent des affections pathologiques chroniques nécessitant de ce fait un traitement régulier et prolongé.

Par conséquent, des inconvénients thérapeutiques qui, de prime abord, paraissent mineurs peuvent au cours du temps, se révéler extrêmement gênants pouvant se solder par un arrêt de la médication et son remplacement par un traitement équivalent.

Des agents neurotropes extrêmement intéressants ont été décrits dans le brevet français No 2.276.036. Il s'agit notamment d'acides dialkylpropioniques, dialkylbutyriques et dialkylvalériques ainsi que leurs sels de métaux alcalins lesquels y sont présentés comme possédant des propriétés anticonvulsivante, antianoxique et inhibitrice compétitive de la transaminase $\gamma$-aminobutyrique $\alpha$-cétoglutarique.

Bien que ces produits soient de valeur indéniable, leur dose thérapeutique journalière chez l'homme est relativement élevée de l'ordre de 1000 mg et même davantage.

Il est évident qu'à pareilles doses, la possibilité d'effets secondaires indésirables est incontestable et doit être prise en considération de manière scrupuleuse.

Par exemple, l'administration prolongée de tels composés pourrait être compromise par suite de désordres hépatiques ou autres.

D'autre part, les dérivés de ce brevet français qu'ils soient administrés par voie orale sous forme d'acide ou de sel de métal alcalin seront présents sous forme d'acide dans le tractus gastro-intestinal ce qui pourrait engendrer des irritations à ce niveau.

Il est bien connu qu'un tel inconvénient peut être évité en utilisant par exemple des formes d'administration entériques par exemple des comprimés recouverts d'un film gastro-résistant.

Dans le cas présent, l'adjonction d'ingrédients supplémentaires sous forme d'un film d'enrobage augmente dans des proportions critiques la taille du comprimé. Celle-ci déjà importante au départ par suite de la quantité élevée de principe actif y incorporé devient telle que l'administration au patient devient pénible surtout s'il s'agit de jeunes enfants ou de personnes âgées.

Ces inconvénients pourraient être évités ou du moins fortement atténués en utilisant soit une quantité moindre de principe actif par unité d'administration, soit une posologie journalière plus faible que celle préconisée pour les composés du brevet français No 2.276.036.

Il devenait impératif, par conséquent, de pouvoir disposer d'agents pharmacologiques dont les propriétés neurotropes se manifesteraient à des doses inférieures à celles des composés du brevet français en question et ce, en vue de diminuer la taille des unités d'administration, le nombre d'unités d'administration par prise et le risque potentiel d'effets secondaires indésirables.

Or, on a trouvé de manière surprenante qu'en remplaçant l'atome de métal alcalin par exemple l'atome de sodium présent chez les sels métalliques du brevet français No 2.276.036 par un atome de métal alcalino-terreux par exemple un atome de calcium ou de magnésium, on augmente d'une manière très significative l'activité anticonvulsivante des sels de ce brevet français.

Ainsi des études pharmacologiques ont montré que cette augmentation d'activité anticonvulsivante peut atteindre de 20 à 45% de l'activité des sels de métaux alcalins correspondants, ce qui est totalement imprévisible d'après l'état de la technique.

En effet, une étude détaillée entreprise avec le di-n-propylacétate de sodium ou n-propyl-2 pentanoate de sodium, agent antiépileptique bien connu, a révélé qu'à des doses identiques, le sel de calcium et le sel de magnésium correspondants présentent des potentialités anti-convulsivantes sensiblement équivalentes à celles du di-n-propylacétate de sodium et même légèrement inférieures.

En outre, on a trouvé de manière surprenante, que le n-propyl-5 octanamide, tout en présentant une bonne activité anticonvulsivante, a révélé un taux d'activité antianoxique de 4 à 8 fois supérieur à ceux enregistrés pour le n-propyl-3 hexanamide et le n-propyl-4 heptanamide, produits déjà connus d'après Eur. Jour. Med. Chem., Chimica Therapeutica vol. X, sept., oct. 1975 n° 5 p. 453—462 pour leur activité anticonvulsivante.

Qui plus est, des composés de l'invention, notamment des sels de métal alcalino-terreux se sont eux-mêmes révélés moins toxiques que les sels de métal alcalin correspondants du brevet français No 2.276.036.

L'ensemble de ces propriétés est susceptible de rendre les composés de l'invention utiles pour le traitement de différentes variétés de troubles du système nerveux central et de désordres relatifs à la neuropsychiatrie tout en évitant les inconvénients des dérivés du brevet français en question.

Comme exemple de tels troubles neurologiques centraux ou de désordres provoqués par un dérèglement du système nerveux central, on peut citer des états et des crises convulsives tels que l'épilepsie, des états choréiques tels que la chorée de Huntington, des troubles de la mémoire, de l'équilibre et de la vigilance ainsi que des vertiges, des chutes de pression artérielle, des céphalées et des états comateux.

Les troubles neuropsychiatriques et les dérèglements du système nerveux central qu'ils soient d'origine ischémique ou pas, constituent un des maux les plus courants à cette époque. De ce fait, il est très difficile pour le praticien de choisir dans la gamme de drogues mises à sa disposition le médicament qui conviendra au cas spécifique à traiter.

Il n'est pas rare qu'en présence, par exemple d'un patient épileptique, le neurologue tatonne en essayant successivement plusieurs drogues pour déceler celle qui sera finalement satisfaisante.

Dans cette optique, les composés de l'invention constitueront une addition précieuse à l'arsenal thérapeutique d'agents dont doit disposer le médicin et à la rigueur, pourront remplacer utilement l'un ou l'autre agent devenu inefficace pour une raison quelconque telle que modification de l'état du malade ou accoutumance.

Les composés de la présente invention qui se sont révélés particulièrement intéressants comme agents neuropsychotropes ou neurotropes, en particulier comme antiépileptiques sont:

le n-propyl-3 hexanoate de calcium
le n-propyl-3 hexanoate de magnésium
le n-propyl-4 heptanoate de calcium
le n-propyl-4 heptanoate de magnésium
le n-propyl-5 octanoate de calcium
le n-propyl-5 octanoate de magnésium
le n-propyl-5 octanamide.

De essais pharmacologiques ont été effectués en vue de mettre en évidence les propriétés pharmacologiques des composés de l'invention. On trouvera ci-dessous les résultats de quelques-uns de ces tests.

I. *Activité anticonvulsivante*

1) Crise à l'acide $\beta$-mercaptopropionique

L'acide $\beta$-mercaptopropionique est un inhibiteur de la glutamate décarboxylase (G.A.D.), enzyme qui catalyse la transformation du glutamate en acide $\gamma$-aminobutyrique (GABA). Son action se traduit donc par un abaissement du taux de GABA et la survenue de crises généralisées tonicocloniques 3 à 4 minutes aprés son administration.

L'acide $\beta$-mercaptopropionique a été administré à des lots de 10 souris mâles, par voie intrapéritonéale, à la dose de 70 mg/kg, 30 minutes après l'administration orale d'une dose du composé à étudier. On note le pourcentage des animaux protégés contre la crise tonique 15 minutes après l'administration de l'acide $\beta$-mercaptopropionique.

On a cité ci-dessous la $DE_{50}$ des composés de l'invention en comparaison avec celle des sels de sodium correspondants:

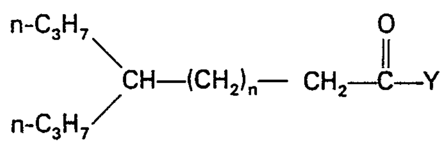

I

| Valeur de Y | n | $DE_{50}$ en mg /kg |
|---|---|---|
| ONa | 0 | 168 |
| $OMg_{1/2}$ | 0 | 145 |
| ONa | 1 | 124 |
| $OCa_{1/2}$ | 1 | 101 |
| ONa | 2 | 273 |
| $OMg_{1/2}$ | 2 | 124 |
| $NH_2$ | 2 | 129 |

Ces résultats montrent nettement que la dose de sel de calcium ou de magnésium de l'invention protégeant 50% des animaux contre la crise à l'acide $\beta$-mercaptopropionique est inférieure à la dose de sel de sodium correspondant nécessaire pour obtenir un taux d'activité équivalent. Dans un autre test comparatif, le di-n-propylacétate de sodium a montré une $DE_{50}$ égale à 175 mg/kg.

2) Crise pentétrazolique

Ce test, pratiqué sur souris par voie orale, est identique à celui décrit dans le brevet français No 2.276.036.

Les $DE_{50}$ obtenues 15 minutes après l'administration du pentétrazol sont reprises ci-dessus comparativement aux résultats trouvés pour les sels de sodium correspondants:

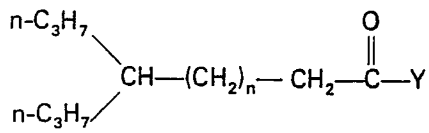

| Valeur de Y | n | $DE_{50}$ en mg /kg |
|---|---|---|
| ONa | 0 | 95 |
| ONa | 1 | 119 |
| ONa | 2 | 172 |
| $OMg_{1/2}$ | 2 | 152 |
| $NH_2$ | 2 | 121 |

II. *Activité antianoxique*

Anoxie par confinement

Des lots de 15 souris mâles sont traités au moyen d'une dose de composé à étudier puis chaque animal est placé dans une enceinte hermétiquement close de 200 $cm^3$. L'animal est considéré comme mort au moment de l'arrêt respiratoire.

On détermine alors le % d'augmentation du temps de survie par rapport à des témoins.

On trouvera ci-dessous des résultats obtenus avec des composés de l'invention à la dose de 75 mg/kg par voie intrapéritonéale comparativement aux n-propyl-3 hexanamide et n-propyl-4 heptanamide:

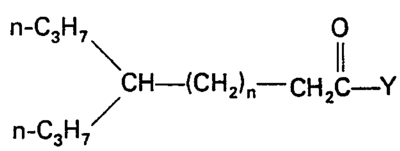

| Valeur de Y | n | % d'augmentation du temps de survie |
|---|---|---|
| $OMg_{1/2}$ | 0 | 36 |
| $OCa_{1/2}$ | 1 | 27 |
| $OMg_{1/2}$ | 2 | 56 |
| $NH_2$ | 2 | 64 |
| n-Propyl-3 hexanamide | | 8 |
| n-Propyl-4 heptanamide | | 15 |

Un test comparatif effectué avec le di-n-propylacétate de sodium n'a montré qu'une augmentation du temps de survie de 32% à la dose de 200 mg/kg également par voie intrapéritonéale.

III. *Toxicité*

Des tests de toxicité aiguë pratiqués sur souris ont montré qu'à la dose de 1000 mg/kg, par voie orale, la mortalité provoquée par le n-propyl-3 hexanoate de magnésium est de 40%.

A titre de comparaison, le n-propyl-3 hexanoate de sodium à la dose de 1000 mg/kg par voie orale provoque déjà 80% de mortalité.

Par conséquent, la dose toxique est plus éloignée de la dose anticonvulsivante dans le cas des composés de l'invention que dans le cas du n-propyl-3 hexanoate de sodium.

De même, les composés de l'invention seront moins susceptibles de provoquer des perturbations des fonctions neuromusculaires aux doses anticonvulsivantes ou antianoxiques que les sels de sodium correspondants.

A cet effet, on a effectué sur souris le test du rota rod selon BOISSIER (Therapie 1958, XIII pp. 1074—1118) lequel permet d'apprécier l'aptitude des animaux à coordonner leurs mouvements sur une tige tournante. Des tests comparatifs ont également été effectués avec les sels de sodium correspondants.

Les résultats suivants sont exprimés en % d'échecs au test aux temps considérés:

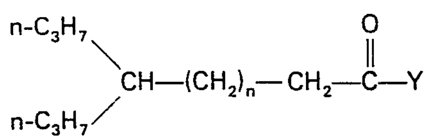

a) 15 minutes après administration de 500 mg/kg par voie orale

| Valeur de Y | n | % d'échecs |
|---|---|---|
| ONa | 1 | 40 |
| $OCa_{1/2}$ | 1 | 20 |
| ONa | 2 | 20 |
| $OMg_{1/2}$ | 2 | 12 |

b) 30 minutes après administration de 500 mg/kg par voie orale

| Valeur de Y | n | % d'échecs |
|---|---|---|
| ONa | 0 | 30 |
| ONa | 1 | 40 |
| $OCa_{1/2}$ | 1 | 20 |

c) 15 minutes après administration de 100 mg/kg par voie intrapéritonéale

| Valeur de Y | n | % d'échecs |
|---|---|---|
| ONa | 1 | 30 |
| $OCa_{1/2}$ | 1 | 0 |

d) 30 minutes après administration de 100 mg/kg par voie intrapéritonéale

| Valeur de Y | n | % d'échecs |
|---|---|---|
| ONa | 2 | 100 |
| $OMg_{1/2}$ | 2 | 70 |
| $NH_2$ | 2 | 50 |

Les compositions thérapeutiques selon l'invention peuvent être présentées sous toute forme convenant à l'administration en thérapie humaine ou vétérinaire. Pour ce qui concerne les unités d'administration, celles-ci peuvent prendre la forme, par exemple, d'une comprimé dragéifié ou non, d'une capsule, d'une gélule, d'une suspension ou d'un sirop pour l'administration orale.

L'unité d'administration comprendra dans ce cas, par exemple, de 100 à 300 mg de principe actif.

Les compositions thérapeutiques de l'invention peuvent également être présentées sous forme d'un suppositoire contenant, par exemple de 200 à 600 mg de principe actif par unité d'administration pour l'administration rectale, ou sous forme d'une solution ou d'une suspension injectable contenant, par exemple, de 50 à 200 mg de principe actif.

Suivant la voie d'administration choisie, les compositions thérapeutiques de l'invention seront préparées en mettant en association au moins un des composés de l'invention avec un véhicule pharmaceutique ou un excipient approprié, ceux-ci pouvant être constitués par exemple d'au moins un ingrédient sélectionné parmi les substances suivantes: talc, stéarate de magnésium, lactose, saccharose, silice colloïdale, carboxyméthylcellulose, amidon de blé ou de maïs, kaolin, levilite, mannitol, beurre de cacao.

La préparation des composés de l'invention ainsi que des compositions thérapeutiques les contenant est illustrée par les Exemples non limitatifs suivants:

Exemple 1

*Preparation du n-propyl-3 hexanoate de magnésium*

a) n-Propyl-2 pentanoate de méthyle

Dans un ballon, parfaitement sec, on introduit, à température ambiante, 142,8 g de chlorure de thionyle et 2 ml de N,N-diméthylformamide. Sous agitation, on introduit progressivement 144,2 g (1 mole) d'acide n-propyl-2 pentanoïque tout en maintenant le mélange à température ambiante. On maintient la température pendant 90 à 120 minutes à température ambiante après la fin de l'introduction puis on porte la masse à 70°C jusqu'à élimination des gaz. On ramène la température à l'ambiante et on introduit progressivement 96 g de méthanol. On porte au reflux, on maintient 60 minutes sous reflux total, on refroidit à température ambiante et on hydrolyse par ajout de 400 g d'eau distillée. On décante la phase aqueuse et on lave la couche organique successivement par une solution aqueuse de bicarbonate de sodium à 5% jusqu'à pH=8—9 et par de l'eau distillée jusqu'à neutralité. On décante au maximum, on sèche et on distille sous vide au bain marie (55 ± 5°C) au moyen d'un évaporateur rotatif (p=2666 Pa)

Rendement: 95%.

De cette manière, on obtient le n-propyl-2 pentanoate de méthyle sous forme brute.

En utilisant le même procédé que décrit ci-dessus, on a préparé le n-propyl-3 hexanoate de méthyle avec un rendement de 96%.

b) n-Propyl-2 pentanol

Dans un ballon, parfaitement sec, on introduit à température ambiante 800 à 850 ml de tétrahydrofuranne anhydre, 81 g (1,5 mole) de borohydrure de potassium et 63,75 g (1,5 mole) de chlorure de lithium. On maintient sous agitation pendant 30 minutes puis on introduit, en une fois, 158,2 g (1 mole) de n-propyl-2 pentanoate de méthyle brut. On porte le mélange au reflux et on l'y maintient pendant 4 heures. On refroidit à température ambiante et on introduit progressivement 180 à 200 ml d'acide acétique glacial tout en maintenant la température ⩽ 40°C et de façon à obtenir un pH=5—6. On hydrolyse ensuite avec 1500 g d'eau épurée, on décante la phase organique et on extrait la phase aqueuse avec deux fractions de 500 ml d'acétate d'éthyle. On réunit les phases organiques et on les lave par 2 fractions de 500 ml d'eau distillée jusqu'à neutrialité. On sèche sur fulfate de sodium et on élimine le solvant avec un évaporateur rotatif. On rectifie l'alcool brut à 110°C sous vide (p=6666—7332 Pa).

De cette manière, on obtient le n-propyl-2 pentanol.

Rendement: 86%.

En utilisant le même procédé que décrit ci-dessus, on a préparé le n-propyl-3 hexanol avec un rendement de 81%. Ce produit a été rectifié à 130°C sous 50 mm Hg.

c) Benzènesulfonate-1 n-propyl-2 pentane

Dans un ballon parfaitement sec; on introduit à température ordinaire 130,2 g (1 mole) de n-propyl-2 pentanol et 316,4 g (4 moles) de pyridine anhydre.

Sous agitation, on introduit progressivement, à température ⩽ 20°C, 194,26 g (1,1 mole) de benzènesulfochlorure.

On maintient durant 5 heurs à température ambiante puis on hydrolyse par ajout de 800 g d'eau épurée et 240 ml d'acide chlorhydrique à 36%. On extrait par 3 fractions de 200 ml d'éther éthylique, on réunit les fractions organiques et on les lave avec 300 g d'eau épurée jusqu'à neutralité. On sèche sur sulfate de sodium et on élimine le solvant sous vide à la température de 55°C ± 5°C au moyen d'un évaporateur rotatif.

De cette manière, on obtient le benzènesulfonate-1 n-propyl-2 pentane brut sous forme d'une huile.

Rendement: 82,5%.

En utilisant le même procédé que décrit ci-dessus, on a préparé le benzènesulfonate-1 n-propyl-3 hexane avec un rendement de 84%.

d) n-Propyl-3 hexanenitrile

Dans un ballon on introduit, à température ambiante, 550 à 600 ml de diméthylsulfoxide, 270,3 g (1 mole) de benzènesulfonate-1 n-propyl-2 pentane et 73,5 g (1,5 mole) de cyanure de sodium.

Sous agitation, on porte la masse aś 90°C, on la maintient pendant 90 minutes à cette température et on refroidit à température ambiante. On hydrolyse avec 1650 à 1800 ml d'eau épurée et on extrait avec 3 fractions de 700 ml d'éther éthylique. On réunit les extraits éthérés, on les sèche sur sulfate de sodium et on élimine le solvant avec un évaporateur rotatif. On rectifie ensuite le nitrile sous vide à une pression de 1600 á 2000 Pa et à une température de 75—80°C.

De cette manière, on obtient le n-propyl-3 hexanenitrile.

Rendement: 92%.

En utilisant le même procédé que décrit précédemment, on a préparé le n-propyl-4-heptanenitrile avec un rendement de 95,4%.

Ce produit a été rectifié à 100—104°C sous 2000 Pa.

e) n-Propyl-3 hexanamide

Dans un ballon, on introduit, à température ambiante, 71 g d'eau épurée et 421 g d'acide sulfurique à 95%, ce qui constitue une solution de 500 g d'acide sulfurique à 80%. Sous agitation, on introduit 139,2 g (1 mole) de n-propyl-3 hexanenitrile. On porte le mélange à 80° et on le maintient à cette température pendant 2 heures. On le refroidit à température ambiante puis on hydrolyse avec 1500 g d'eau épurée durant 1 heure sous agitation. On essore le produit brut et on le reprend sous agitation durant 1 heure à température ambiante dans 1500 g d'eau épurée. On essore de nouveau, on lave, on sèche à poids constant en étuve à vide.

La purification de l'amide brut ainsi obtenu a été réalisée comme suit:

On dissout par chauffage au reflux 157,3 g (1 mole) d'amide brut dans 157 ml de dichloréthane. On filtre à chaud, on rince par la quantité minimum de dichloréthane chaud et on laisse cristalliser par refroidissement sous agitation. On essore après 2 heures de maintien à −5°/−10°C, on rince avec la quantité minimum de dichloréthane glacé et on sèche à poids constant en étuve à vide à 40°C.

De cette mainère, on obtient le n-propyl-3 hexanamide.

P.F. 112°C.

En utilisant le même procédé que précédemment, on a préparé le n-propyl-4 heptanamide.

P.F.: 61°C.

f) n-Propyl-3 hexanoate de magnésium

Dans un ballon, on introduit 79 g d'eau épurée et 421 g d'acide sulfurique à 95% ce qui équivaut à 500 g d'acide sulfurique à 80% puis, sous agitation 157,2 g (1 mole) de n-propyl-3 hexanamide brut. On porte le mélange à 80—85°C pendant une heure, on refroidit à 50°C et on introduit à 55°C ± 5° une solution de 200 g d'eau épurée et 98 g de nitrite de sodium. On règle la vitesse d'introduction sur la température et l'absence pratiquement totale de fumées rouges d'oxyde d'azote.

Dès la fin d'introduction, on refroidit à température ambiante et on dilue par 300 g d'eau épurée. On extrait le milieu réactionnel avec trois fractions de 200 ml d'éther éthylique, on réunit les extraits éthérés et on extrait les sels de sodium avec une solution de 44 g d'hydroxyde de sodium dans 400 g d'eau épurée. On lave la phase aqueuse avec deux fractions de 200 ml d'éther éthylique et on relargue l'acide de son sel par 110 ml d'acide chlorhydrique à 36%. On extrait avec trois fractions de 200 ml d'éther éthylique, on réunit les extraits éthérés et on les sèche sur sulfate de sodium. On élimine le solvant sous vide au moyen d'un évaporateur rotatif et on rectifie l'acide brut sous vide (p ≃ 133,3 Pa) à la température de 102°C, ce qui fournit 78,8% d'acide n-propyl-3 hexanoïque.

On agite ensuite pendant 4 heures à température ambiante 161,4 g (1,02 mole) d'acide n-propyl-3 hexanoïque, 29,15 g (0,5 mole) d'oxyde de magnésium et 500 g d'eau épurée. Le sel de magnésium ainsi formé précipite sous forme pâteuse.

On porte l'ensemble réactionnel à sec avec un évaporateur rotatif sous une pression de 2666,4 Pa et à la température de 55°C ± 5°.

De cette manière, on obtient le n-propyl-3 hexanoate de magnésium sous forme d'une poudre blanche.

Rendement: 98%.

Solution aqueuse à 2% légèrement opalescente et de pH=9,4.

En utilisant le même procédé que précédemment on a préparé les composés suivants:

Composé

n-Propyl-4 heptanoate de magnésium
Rendement: 96%
n-Propyl-5 octanoate de magnésium
(poudre très légèrement crème)
Rendement: 95%
Solubilité dans l'eau: ≃0,1%
Bonne solubilité dans les solvants organiques.

## Exemple 2

*Préparation du n-propyl-4 heptanoate de calcium*

Dans un ballon, on introduit 500 g d'eau et 28 g (0,5 mole) d'oxyde de calcium. Sous agitation, on porte la masse à 70°C et on introduit, à cette température 172,3 g (1 mole) d'acide n-propyl-4 heptanoïque.

On maintient durant 2 heures à 70°C et le sel de calcium précipite sous forme pâteuse. On met à sec le milieu réactionnel avec un évaporateur rotatif et on sèche à poids constant en étuve à vide à 80°C.

De cette manière, on obtient le n-propyl-4 heptanoate de calcium sous forme d'une poudre blanche.

Rendement: 97%

Ce produit se dissout très lentement dans l'eau dans les proportions de 100 g de sel pour 100 ml d'eau.

De la même manière que celle décrite précédemment, on a préparé les composés suivants:

Composé

n-Propyl-3 hexanoate de calcium
Rendement: 96%
n-Propyl-5 octanoate de calcium
Rendement: 95,5%

## Exemple 3

*Préparation du n-propyl-5 octanamide*

a) n-Propyl-3 hexanoylmalonate de diéthyle

Dans un ballon parfaitement sec, on introduit, à température ambiante, 176 g de malonate de diéthyle et 400 ml de N,N-diméthylformamide. Tout en maintenant le milieu réactionnel à la température ambiante, on introduit progressivement 48 g d'hydrure de sodium à 50%. On attend la cessation du dégagement gazeux puis on introduit rapidement, en une fois, 284,4 g de benzènesulfonate-1 n-propyl-3 hexane. On porte la masse à 90°C ± 10° pendant 1 heure, on refroidit à température ambiante et on hydrolyse par ajout de 1200 g d'eau épurée. On extrait l'ester malonique par 3 fractions de 400 ml d'éther éthylique et on réunit les phases éthérées. On les lave à l'eau, on les

sèche sur sulfate de sodium et on élimine le solvant sous pression réduite avec un évaporateur rotatif. On étête l'ester brut sous pression réduite de 266,6 Pa jusqu'à 100°C dans la masse.

De cette manière, on recueille le n-propyl-3 hexanoylmalonate de diéthyle.

Rendement: 81%.

b) Acide n-propyl-5 octanoïque

Dans un ballon, on introduit 280 g d'une solution aqueuse d'hydroxyde de potassium à 50%, 286 g de n-propyl-3 hexanoylmalonate de diéthyle et 100 ml de méthanol. Sous agitation, on porte le mélange au reflux et on l'y maintient pendant 1 heure. On élimine les alcools et on dilue par 400 g d'eau épurée.

On extrait les insaponifiés avec 3 fractions de 200 ml d'éther éthylique et on libère l'acide malonique et son sel dipotassique par 250 ml d'acide chlorhydrique à 36%.

On extrait par 3 fractions de 200 ml d'éther éthylique et on réunit les extraits éthérés. On sèche sur sulfate de sodium et on élimine le solvent sous pression réduite. On charge le dérivé d'acide malonique brut dans un ballon de 250 ml et sous agitation et à pression normale, on porte la masse à 180—190°C jusqu'à fin de décarboxylation.

On laisse refroidir vers 100°C et on rectifie, sous pression réduite, l'acide n-propyl-5 octanoïque obtenu. On recueille la fraction bouillant à 122°C sous 133,3 Pa et on purifie l'acide ainsi isolé par passage au sel de sodium en solution aqueuse, extraction des impuretés à l'éther éthylique et libération de l'acide désiré par l'acide chlorhydrique à 36% suivie d'une nouvelle rectification. On recueille la fraction bouillant à 123°C/133,3 Pa.

De cette manière, on obtient l'acide n-propyl-5 octanoïque avec un rendement de 50% à partir du dérivé malonique.

Spectre infra-rouge: OH à 3500—2300 $cm^{-1}$ (m, l)
C=O à 1710 $cm^{-1}$ (F)

c) n-Propyl-5 octanamide

Dans un ballon, on introduit 300 ml de toluène anhydre. On glace à température inférieure ou égale à −10°C et on introduit 40 g (2,35 moles) d'ammoniac. Toujours à cette température, on introduit progressivement une solution de 205 g (1 mole) de chlorure de n-propyl-5 octanoyle brut préparé à partir de l'acide correspondant et de chlorure de thionyle. On fait de nouveau barboter de l'ammoniac et on maintient pendant 30 minutes à température $\leqslant -10$°C.

On ajoute alors 300 g d'eau épurée, on décante après dissolution du chlorure d'ammonium et on lave la couche organique avec 2 fractions de 300 g d'eau épurée jusqu'à pH neutre. On élimine le toluène sous pression réduite d'environ 2666 Pa et à une température de 55°C ± 5°C. On obtient la cristallisation de l'amide par refroidissement. On reprend par 100 ml de n-heptane, on dissout par chauffage et on cristallise sous agitation. On essore après 2 heures de maintien à −10°C et on rince par un minimum d'heptane glacé. On sèche à poids constant en étuve à vide à température ambiante.

De cette manière, on obtient 130 g de n-propyl-5 octanamide sous forme d'une poudre blanche.

Rendement: 70%

P.F.: 44°C.

## Exemple 4

*Préparation du n-propyl-3 hexanoate de calcium*

Dans un ballon, on introduit 450 g d'eau épurée puis, sous agitation, 216 g (3,85 moles) d'oxyde de calcium. On maintient sous agitation pendant 15 minutes à la température du milieu réactionnel (50°C). On introduit alors 5.500 g de n-propanol puis par une ampoule à brome, on ajoute 1.135,5 g (7,18 moles) d'acide n-propyl-3 hexanoïque. On porte au reflux pendant une heure puis on élimine l'insoluble, représenté par de la chaux en excès, au moyen d'une filtration sur Büchner. On rince le ballon et le Büchner avec 150 g de n-propanol puis on charge le filtrat dans un appareil à distiller.

On élimine l'eau présente par azéotropie puis, après, distillation de l'azéotrope, on recueille une suspension de n-propyl-3 hexanoate de calcium dans le milieu. On refroidit à 50°C puis on ajoute 2.000 g d'acétone. On agite, on filtre et on rince le précipité de n-propyl-3 hexanoate de calcium avec de l'acétone. On sèche en étuve ventilée à 105°C jusqu'à poids constant.

De cette manière, on obtient le n-propyl-3 hexanoate de calcium avec un rendement de 88,5%.

## Exemple 5

*Préparation du n-propyl-4 heptanoate de magnésium*

Dans un ballon équipé d'un agitateur, d'un chauffage et d'une superstructure permettant un reflux, on introduit 172 g (1 mole) d'acide n-propyl-4 heptanoïque et 500 ml de benzène. Tout en agitant, on ajoute 16 g d'oxyde de magnésium. On chauffe à 40°C jusqu'à disparition de l'oxyde de magnésium ce qui nécessite 3 heures environ. Après la disparition totale de la magnésie, on laisse le milieu réactionnel revenir à la température ambiante et on ajoute 1.000 ml d'eau épurée. On agite pour solubiliser le sel de magnésium dans la phase aqueuse puis on interrompt l'agitation. On laisse décanter et on sépare la couche aqueuse. On l'extrait à nouveau avec 250 ml de benzène pour parfaire l'élimination de l'acide n-propyl-4 heptanoïque en excès. On traite la solution aqueuse avec 2% de charbon

actif par rapport au poids de n-propyl-4 heptanoate de magnésium et ce par agitation à 50°C pendant 2 heures. On élimine le charbon et on concentre à sec la solution aqueuse dans un évaporateur rotatif.

De cette manière, on recueille 146,4 g de n-propyl-4 heptanoate de magnésium.

Exemple 6

Suivant des techniques pharmaceutiques connues, on a préparé des comprimés contenant les ingrédients suivants:

| Ingredients | mg par comprimé |
| --- | --- |
| n-Propyl-5 octanamide | 100 |
| Mannitol | 69 |
| Amidon de maïs | 60 |
| Silice colloïdale | 12 |
| Stéarate de magnésium | 9 |
| | 250 |

**Revendications**

1. Dérivés d'acide ω-dialkylalkanoïque correspondant à la formule générale:

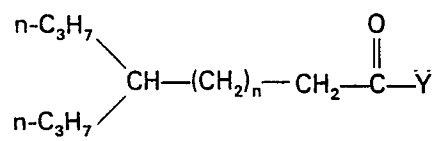

dans laquelle Y représente un radical $NH_2$ ou un radical $OM_{1/2}$ dans lequel M représente un atome de métal alcalino-terreux et n représente l'une des valeurs 0, 1 ou 2, à condition que lorsque Y représente $NH_2$, n représente 2.

2. n-Propyl-3 hexanoate de calcium ou de magnésium.
3. n-Propyl-4 heptanoate de calcium ou de magnésium.
4. n-Propyl-5 octanoate de calcium ou de magnésium.
5. n-Propyl-5 octanamide.
6. Procédé de préparation de dérivés d'acide ω-dialkylalkanoïque de formule générale:

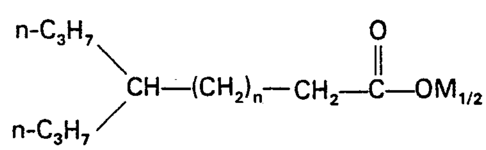

dans laquelle M représente un atome de métal alcalino-terreux et n représente 0, 1 ou 2, caractérisé en ce que l'on fait réagir, dans un solvant approprié et à une température comprise entre la température ambiante et 70°C, un acide de formule générale:

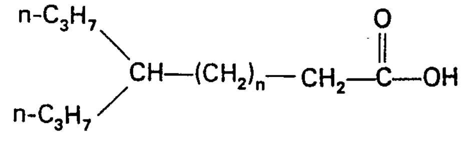

dans laquelle n a la même signification que précédemment, avec un oxyde de métal alcalino-terreux.

7. Procédé selon 6 caractérisé en ce que le solvant est l'eau, le n-propanol ou le benzène.
8. Procédé de préparation du n-propyl-5 octanamide de formule:

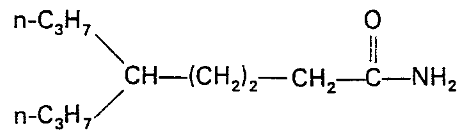

caractérisé en ce que l'on fait réagir l'ammoniac avec le chlorure de n-propyl-5 octanoyle la réaction ayant lieu dans un solvant approprié et à une température comprise entre −15°C et 0°C.

9. Procédé selon 8 caractérisé en ce que le solvant est le toluène.

10. Medicaments utiles dans le traitement de troubles neurologiques centraux consécutifs ou non à l'ischémie cérébrale ainsi que de troubles relevant de la neuropsychiatrie, caractérisés en ce qu'ils contiennent à titre de principe actif un dérivé d'acide ω-dialkylalkanoïque de formule générale:

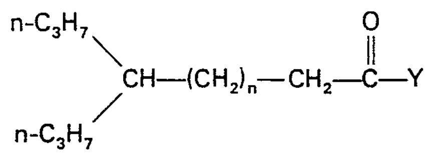

dans laquelle Y représente un radical $NH_2$ ou un radical $OM_{1/2}$ dans lequel M représente un atome de métal alcalino-terreux et n représente l'une des valeurs 0, 1 ou 2, à condition que lorsque Y représente $NH_2$, n représente 2.

11. Medicaments selon 10, caractérisés en ce que le métal alcalino-terreux est le calcium ou le magnésium.

**Claims**

1. ω-Dialkylalkanoic acid derivatives corresponding to the general formula:

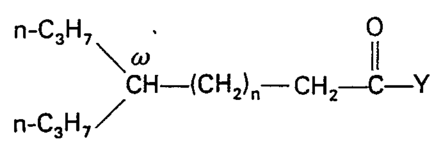

wherein Y represents a radical $NH_2$ or a radical $OM_{1/2}$ in which M represents an alkaline earth metal atom and n represents one of the values 0, 1 or 2, with the proviso that when Y represents $NH_2$, n represents 2.

2. Calcium or magnesium 3-n-propyl-hexanoate.
3. Calcium or magnesium 4-n-propyl-heptanoate.
4. Calcium or magnesium 5-n-propyl-octanoate.
5. 5-n-Propyl-octanamide.
6. Process for preparing ω-dialkylalkanoic acid derivatives of general formula:

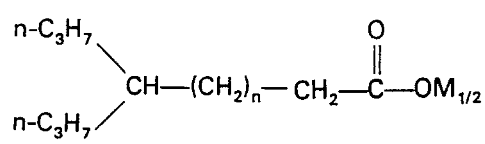

in which M represents an alkaline earth metal atom and n represents 0, 1 or 2, whereby an acid of general formula:

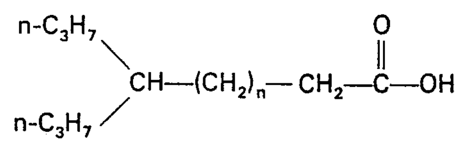

in which n has the same meaning as given above, is reacted in an appropriate solvent and at a temperature between room-temperature and 70°C, with an alkaline earth metal oxide.

7. Process according to Claim 6 wherein the solvent is water, n-propanol or benzene.
8. Process for preparing 5-n-propyl-octanamide of formula:

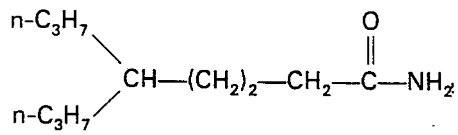

whereby ammonia is reacted with 5-n-propyl-octanoyl chloride, the reaction taking place in an appropriate solvent and at a temperature between −15°C and 0°C.

9. Process according to Claim 8 wherein the solvent is toluene.

10. Medicaments useful in the treatment of central neurological disorders whether resulting or not from cerebral ischemia, and of disorders relating to the field of neuropsychiatry, said medicaments containing, as active principle, an $\omega$-dialkylalkanoic acid derivative corresponding to the general formula:

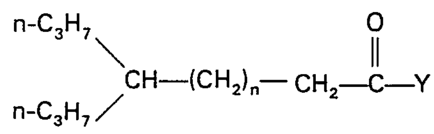

in which Y represents a radical $NH_2$ or a radical $OM_{1/2}$ in which M represents an alkaline earth metal atom and n represents one of the values 9, 1 or 2, with the proviso that when Y represents $NH_2$, n represents 2.

11. Medicaments according to Claim 10, wherein the alkaline earth metal atom is calcium or magnesium.

**Patentansprüche**

1. $\omega$-Dialkylalkansäure-Derivate der allgemeinen Formel:

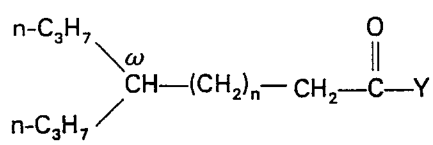

worin Y eine $NH_2$-Gruppe oder eine Gruppe $OM_{1/2}$, wobei M ein Erkalkalimetall-Atom ist, bedeutet, und n einen Wert von 0, 1 oder 2 hat unter der Voraussetzung, daß n=2 ist, wenn Y eine $NH_2$-Gruppe bedeutet.

2. Calcium- oder Magnesium-3-n-propyl-hexanoat.
3. Calcium- oder Magnesium-4-n-propyl-heptanoat.
4. Calcium- oder Magnesium-5-n-propyl-octanoat.
5. 5-n-Propyl-octanamid.
6. Verfahren zur Herstellung von $\omega$-Dialkylalkansäure-Derivaten der allgemeinen Formel

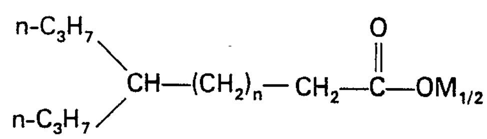

worin M ein Erkalkalimetallatom bedeutet, und n einen Wert 0, 1 oder 2 hat, wobei eine Säure der allgemeinen Formel:

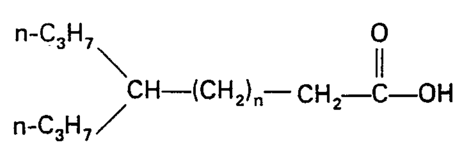

worin n die gleiche Bedeutung, wie oben angegeben, hat, in einem geeigneten Lösungsmittel bei einer Temperatur zwischen Zimmertemperatur und 70°C mit einem Erdalkalimetalloxid umgesetzt wird.

7. Verfahren nach Anspruch 6, worin das Lösungsmittel Wasser, n-Propanol oder Benzol ist.

8. Verfahren zur Herstellung von 5-n-Propyl-octanamid der Formel

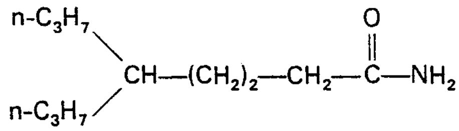

wobei Ammoniak mit 5-n-Propyl-octanoylchlorid umgesetzt wird und die Reaktion in einem geeigneten Lösungsmittel bei einer Temperatur zwischen −15°C und 0°C stattfindet.

9. Verfahren nach Anspruch 8, worin das Lösungsmittel Toluol ist.

10. Medikamente, die zur Behandlung von Zentralneurologischen Störungen, die gegebenenfalls auf zerebraler Ischämie beruhen, sowie von Störungen auf dem Gebiet der Neuropsychiatrie geeignet sind, wobei diese Medikament als aktiven Bestandteil ein $\omega$-Dialkylalkansäure-Derivat der allgemeinen Formel:

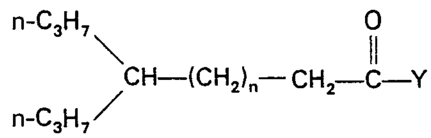

worin Y eine $NH_2$-Gruppe oder eine Gruppe $OM_{1/2}$, wobei M ein Erdalkalimetallatom ist, bedeutet, und n einen Wert von 0, 1 oder 2 hat unter der Voraussetzung, daß n=2 ist, wenn Y eine $NH_2$-Gruppe bedeutet, enthalten.

11. Medikaments nach Anspruch 10, worin das Erdalkalimetallatom Calcium oder Magnesium ist.